# EUROPÄISCHE PATENTSCHRIFT

(11) **EP 0 962 192 B1**
(45) Veröffentlichungstag und Bekanntmachung des Hinweises auf die Patenterteilung: **03.08.2005**
(21) Anmeldenummer: 99109700.7
(22) Anmeldetag: 17.05.1999
(51) Int. Cl.: A61C 3/03, A61B 17/32

(54) **Medizinisches oder dentalmedizinisches Instrument zur spanabhebenden Bearbeitung von Körpergewebe und Werkzeug für ein solches Instrument**
Medical or dental instrument for cutting body tissue and tool therefore
Instrument médical ou dentaire, destiné à couper des tissue corporels ainsi que son outil

(30) Priorität: 05.06.1998 DE 19825261
(43) Veröffentlichungstag der Anmeldung: 08.12.1999
(73) Patentinhaber: Kaltenbach & Voigt GmbH & Co. KG, 88400 Biberach/Riss (DE)
(72) Erfinder: Hugo, Burkhard, Dr., 97265 Hettstadt (DE)
(74) Vertreter: Schmidt-Evers, Jürgen, Dipl.-Ing.

(56) Entgegenhaltungen:
- EP-A- 0 106 632
- EP-A- 0 360 161
- WO-A-94/16640
- DE-A- 19 541 032
- DE-U- 29 509 919
- US-A- 4 353 696

## Beschreibung

Die Erfindung betrifft ein medizinisches oder dental medizinisches Instrument und/oder ein Werkzeug nach dem Oberbegriff des Anspruchs 1 bzw. 2.

Zur spanabhebenden Bearbeitung von Hart- oder Weichgeweben des menschlichen oder tierischen Körpers oder künstlichen Teilen davon, wie z.B. Prothesen, sind bereits verschiedene Instrumente und zugehörige Werkzeuge bekannt geworden. Hierbei kann es sich sowohl um Werkzeuge für eine Oberflächenbehandlung als auch um Werkzeuge zur Ausarbeitung einer Kavität oder um Trennwerkzeuge handeln. Grundsätzlich kann man die Arbeitsbewegungen eines vorliegenden Werkzeugs und zugehörige Instrumente mit Antrieben in drei Gruppen unterteilen, nämlich Rotationsbewegungen, Longitudinalbewegungen und Schwingbewegungen. Eine Rotationsbewegung findet bei üblichen Bohrern oder Fräsern sowie bei Kreissägen statt. Longitudinalbewegungen werden zur Oberflächenbearbeitung angewandt, wie es bei Feilen der Fall ist, wobei die Oberflächenbearbeitung in einer Kavität, z.B. in einem Wurzelkanal, oder an einer Außenfläche stattfinden kann, z.B. an der Oberfläche eines Zahns oder Knochens. Es sind auch Kombinationen von vorgenannten Bewegungen möglich und bei Instrumenten und Werkzeugen zur Aufbereitung von Zahnwurzelkanälen auch bereits vorgeschlagen worden. Eine besondere Arbeitsbewegung ist eine Schwingbewegung, bei der es sich um eine longitudinale Bewegung, um eine in einer Ebene umlaufende, z.B. auf einer kreisförmigen oder elliptischen Bahn umlaufende, Bewegung, oder um eine räumliche Bewegung handeln kann. Wesentliche Merkmale einer solchen Schwingbewegung sind eine hohe Frequenz und kleine Amplitude.

Instrumente und Werkzeuge der zuletzt angegebenen Art sind für dentale Zwecke in der WO 96/14024 beschrieben.

Ein dentalmedizinisches Instrument und ein Werkzeug für ein dentalmedizinisches Instrument der eingangs angegebenen Arten sind in der EP 0 360 161 A beschrieben. Bei diesem vorbekannten dentalmedizinischen Instrument handelt es sich um ein Poliergerät mit einem hakenförmigen Polierwerkzeug, dessen freier Endbereich allseitig eine abrasive Arbeitsfläche aufweist, die durch eine Beschichtung mit Diamantstaub gebildet ist. Der hakenförmige Endbereich des Werkzeugs weist dabei eine dreieckförmige oder ellipsenförmige Querschnittsform jeweils mit konvex gerundeten Seitenflächen auf.

Der Erfindung liegt die Aufgabe zugrunde, ein Instrument und/oder ein Werkzeug der eingangs angegebenen Arten so auszugestalten, daß sein Einsatzbereich vergrößert wird.

Diese Aufgabe wird durch die Merkmale des Anspruchs 1 oder 2 gelöst.

Das erfindungsgemäße Instrument zeichnet sich dadurch aus, daß es einen Werkzeugkörper in Form eines Blattes aufweist, das schmalseitig die Arbeitsfläche aufweist. Im Gegensatz zur bekannten Ausgestaltung führt das erfindungsgemäße Werkzeug die spanabhebende Bearbeitung auf einem dünnen bzw. linienförmigen Streifen durch, dessen Breite durch die Dicke des Blattes vorgegeben ist. Hierdurch ist das Werkzeug nicht nur leistungsfähig, weil lange Schnitte bei verhältnismäßig geringem Spananfall ausgeführt werden können, sondern es ist auch möglich, nach der Durchführung eines Schnittes einen oder mehrere zu dem oder den vorherigen Schnitten quer verlaufende Schnitte auszuführen, so daß ein Teil des zu bearbeitenden Gegenstandes ausgeschnitten werden kann. Dabei liegt der Erfindung die Erkenntnis zugrunde, daß aufgrund der verhältnismäßig geringen Amplitudengröße der Schwingungen das Blatt des Werkzeugs in den zu bearbeitenden Gegenstand eingesenkt werden kann, ohne das die Funktion des Werkzeugs wesentlich beeinträchtigt oder aufgehoben wird wie es bei üblichen Hubsägen der Fall ist, die nicht in den zu bearbeitenden Gegenstand eingesenkt werden können, weil dadurch der relativ große Hub des Sägeblattes aufgehoben wird und die Säge funktionsuntüchtig wird. Im Gegensatz dazu bleibt beim erfindungsgemäßen Instrument aufgrund der kleinen Schwingungsgrößen der Werkzeugkörper bzw. das Blatt auch dann unbeeinträchtigt, wenn der Werkzeugkörper bzw. das Blatt in den zu bearbeitenden Werkstoff eingesenkt wird. Deshalb eignet sich das erfindungsgemäße Instrument nicht nur zum Trennen eines Gegenstandes, der kleiner ist als die Länge des Werkzeugs, wie es bei Sägen üblich ist, sondern der Werkzeugkörper bzw. das Blatt kann kleiner als die Abmessung des Gegenstandes bemessen sein und kann auch in den zu bearbeitenden Gegenstand eingesenkt werden.

Die Erfindung bezieht sich auch auf ein medizinisches oder dentalmedizinisches Instrument zur spanabhebenden Ausarbeitung von Ausnehmungsöffnungen für Implantate in einem Körpergewebe des menschlichen oder tierischen Körpers.

Bisher war es bekannt, die Ausnehmungsöffnungen für Implantate durch ein rotierendes Werkzeug herzustellen und Implantate entsprechender Querschnittsform in die Aufnahmeöffnungen einzusetzen.

Diese bekannte Vorgehensweise ist nachteilig, weil zum einen rotierende Werkzeuge während der spanabhebenden Ausarbeitung zum Verlaufen neigen und außerdem durch rotierende Werkzeuge eine verhältnismäßig große Querschnittsgröße für das Aufnahmeloch und für das Implantat vorgegeben ist. Außerdem ist eine rotationssymmetrische Querschnittsform für ein Implantat hinsichtlich der Implantatverankerung und der Festigkeit der Einbettung nachteilig bzw. den Anwendungsbereich einschränkend. Die vorgenannten Schwierigkeiten bestehen insbesondere dann, wenn eine Aufnahmeöffnung nur teilweise in den vorhandenen Kieferknochen hineinreicht, z.B. dann, wenn es gleichzeitig in die Zahnwurzeltasche eines vorherigen Zahnes hineinreicht. Längsovale Implantatquerschnitte, wie sie der natürlichen Zahnwurzel, z. B. im Oberkieferfrontzahnbereich, entsprechen, sind bei rotierender Präparation nicht möglich. Auch lassen sich nierenförmige Querschnitte, wie sie im Unterkieferzahnbereich auftreten, rotierend nicht präparieren.

Mit dem erfindungsgemäßen Instrument wird eine einfache Plazierung der Aufnahmeöffnung und/oder ein stabiler Sitz für ein Implantat erreicht.

Bei dem erfindungsgemäßen Instrument finden keine Rotationsbewegungen statt. Der Werkzeugkörper des Werkzeugs weist an seiner in die Wirkrichtung des Werkzeugs weisenden Schmalseite eine abrasive Arbeitsfläche auf, die aufgrund des Vibrationsantriebs des Instruments abrasiv wirksam ist und dabei das Gewebe gleichmäßig spanabhebend abträgt. Hierbei läßt sich das Aufnahmeloch in einfacher Weise paßgenau plazieren. Da beim erfindungsgemäßen Instrument keine Rotationsbewegungen stattfinden, lassen sich auch Querschnittsformen verwirklichen, die von einer rotationssymmetrischen Querschnittsform abweichen. Dabei ist es von besonderem Vorteil, eine abgeflachte oder längliche Querschnittsform bezüglich des Gegenstandes länglich anzuordnen. Diese Anordnung ist sehr platzsparend und führt außerdem zu einer stabilen Einbettung des Implantats im Gewebe. Diese Vorteile gelten insbesondere für Implantate des Kiefers, in dessen Bereich im Hinblick auf die anatomischen Gegebenheiten und das Vorhandensein von Nervenkanälen wenig Raum vorhanden ist. Bei einer Anordnung der unrunden Querschnittsform des Aufnahmelochs in bukko-lingualer Richtung läßt sich somit bedeutender Raum gewinnen, Von besonderer Bedeutung ist der Gewinn an Verankerungsfläche, insbesondere dann, wenn die anatomischen Vorraussetzungen keine tiefe Präparation zulassen.

Die Erfindung bezieht sich gemäß Anspruch 2 auch auf ein Werkzeug für ein erfindungsgemäßes Instrument bezüglich der dadurch erzielbaren Vorteile wird auf die vorbeschriebenen Vorteile verwiesen.

Die erfindungsgemäßen Ausgestaltungen eignen sich sowohl für Hart- als auch Weichgewebe wie Knochen oder Fleisch oder Haut.

Das erfindungsgemäße Werkzeug eignet sich für ein längliches, insbesondere gerades oder winkelförmiges, Handstück, das eine im vorderen Endbereich des Handstücks angeordnete lösbare Haltevorrichtung für das Werkzeug aufweist, wobei im Handstück ein Vibrationsantrieb für das Werkzeug angeordnet ist, mit dem hochfrequente, in wenigstens einer Ebene hin und her oder umlaufende oder räumliche Schwingbewegungen auf das Werkzeug übertragbar sind.

In den Unteransprüchen sind Merkmale enthalten, die zu einfachen, kostengünstig herstellbaren, funktionssicheren und leistungsfähigen Ausgestaltungen kleiner Bauweise des Instruments und/oder Werkzeugs führen.

Nachfolgend werden die Erfindung und weitere durch sie erzielbare Vorteile anhand von bevorzugten Ausgestaltungen und vereinfachten Zeichnungen näher erläutert. Es zeigen
- Fig. 1: ein erfindungsgemäßes medizinisches oder dentalmedizinisches Instrument in der Seitenansicht;
- Fig. 2: einen Instrumentenschaft eines abgewandelten Instruments mit einer Haltevorrichtung für ein Werkzeug in abgewandelter Ausgestaltung;
- Fig. 3: ein Werkzeug in abgewandelter Ausgestaltung;
- Fig. 4: den Instrumentenschaft und das Werkzeug nach Fig. 3 in unterschiedlichen Arbeitsstellungen des Werkzeugs;
- Fig. 5: ein Werkzeug in abgewandelter Ausgestaltung;
- Fig. 6: den Instrumentenschaft mit einem darin gehaltenen Werkzeug in perspektivischer Vorderansicht;
- Fig. 7: ein Werkzeug in weiter abgewandelter Ausgestaltung in perspektivischer Ansicht;
- Fig. 8: ein Werkzeug in weiter abgewandelter Ausgestaltung in der Draufsicht;
- Fig. 9: eine Operationsdarstellung mit Operationsschnitten von erfindungsgemäßen Werkzeugen bei einer Zahnwurzelspitzenpräparation;
- Fig. 10: ein weiter abgewandeltes Werkzeug mit einem Instrumentenschaft in der Seitenansicht;
- Fig. 11: das Werkzeug im Schnitt XI-XI in Fig. 10 in einer zwischen zwei einander benachbarten Zähnen befindlichen Arbeitsstellung;
- Fig. 12: ein weiter abgewandeltes Werkzeug in der Seitenansicht;
- Fig. 13: den Schnitt XIII-XIII in Fig. 12.

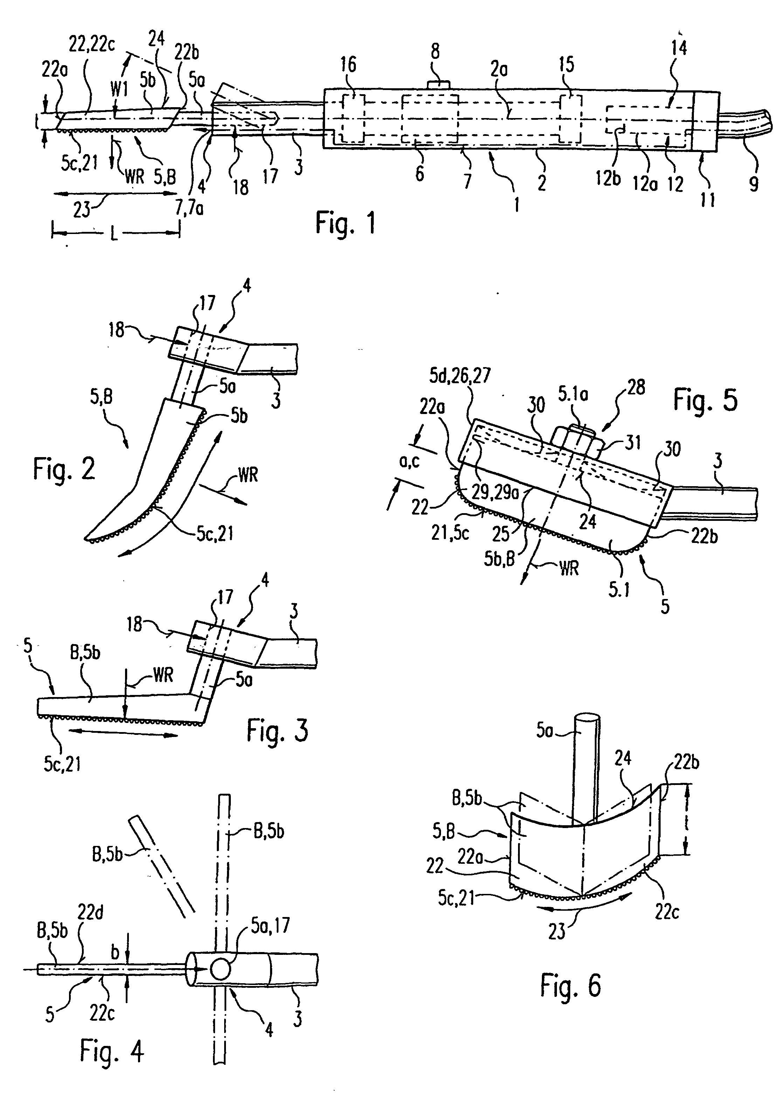

Das in Fig. 1 allgemein mit 1 bezeichnete Instrument eignet sich insbesondere für Knochen-, Zahn- und Kieferbearbeitungen. Die Hauptelemente des Instruments 1 sind ein längliches bzw. stabförmiges Handstück 2, von dessen vorderen Ende ein Handstückschaft 3 absteht, in dessen freien Endbereich eine Haltevorrichtung 4 angeordnet ist, in der ein Werkzeug 5 mit einem Werkzeugschaft 5a und einem davon abstehenden Werkzeugkörper 5b lösbar gehalten ist, ein Vibrationsantrieb 6 für den Handstückschaft 3, der im Handstück 2 angeordnet ist, und vorzugsweise auch eine elektronische Steuereinrichtung zur Vergrößerung oder Verringerung der Antriebsleistung, wobei die Steuereinrichtung im Instrument 1 bzw. Handstück 2 oder auch entfernt davon, z.B. an einem nicht dargestellten Steuergerät oder einem Fußschalter angeordnet sein kann. Vorzugsweise ist zur Einstellung der gewünschten Antriebsleistung ein allgemein mit 8 bezeichnetes Einstellglied vorgesehen, das bei der vorliegenden Ausgestaltung an der Mantelfläche des Handstücks 2 angeordnet ist und dort verschiebbar gelagert ist, jedoch auch vom Handstück 2 oder Instrument 1 entfernt angeordnet sein kann. Das Handstück 2 kann sich gerade erstrecken oder es kann ein sogenanntes Winkelstück mit einer abgewinkelten Griffhülse sein.

Es ist vorteilhaft, eine z.B. externe, vorzugsweise sterile, Kühlmediumzuführung vorzusehen, die sich z.B. an der Werkzeugbefestigung erstrecken kann. Es kann auch eine interne Kühlmediumzuführung vorgesehen sein, die sich durch Befestigungs- oder Versteifungselementen für das Werkzeug erstrecken.

Das Instrument 1 ist durch eine andeutungsweise dargestellte flexible Versorgungsleitung 9 mit einem flexiblen Versorgungsschlauch mit dem Steuergerät verbunden, wobei in oder an der Versorgungsleitung 9 eine oder mehrere Medienleitungen 7 zur Versorgung des Instruments 1 mit Energie und Behandlungs- und/oder Bearbeitungsmedien verlaufen.

Bei der vorliegenden Ausgestaltung besteht das Instrument 1 aus dem ein vorderes Instrumententeil bildenden Handstück 2 und einem ein hinteres Instrumententeil bildendes Anschlußstück 11, das an seinem hinteren Ende mit der flexiblen Versorgungsleitung 9 verbunden ist und durch eine Schnellschlußkupplung 12, insbesondere eine Steck- oder eine Schraubkupplung, mit dem Handstück 2 lösbar verbunden ist. Bei der Schnellschlußkupplung 12 handelt es sich vorzugsweise um eine solche, die im gekuppelten Zustand ein Drehen des Handstücks 2 um seine Längsmittelachse 2a und dabei den Durchgang des oder der vorhandenen Medien gewährleistet. Bei der vorliegenden Ausgestaltung ist eine Steckkupplung mit einem zylindrischen oder stufenzylindrischen Kupplungszapfen 12a und eine ihn drehbar aufnehmende Kupplungsausnehmung 12b vorgesehen, wobei beim vorliegenden Ausführungsbeispiel der Kupplungszapfen 12a vom Anschlußstück 11 nach vorne absteht und die Kupplungsausnehmung 12b nach hinten aus dem Handstück 2 ausmündet. Durch eine ansich bekannte lösbare, insbesondere manuell überdrückbare elastische Sicherungsvorrichtung 14 ist im gekuppelten Zustand ein unbeabsichtigtes Lösen der Steckkupplung verhindert. Für einen Trennvorgang läßt sich die mit einem elastisch vorgespannten Sicherungselement wirksame Sicherungsvorrichtung 14 handhabungsfreundlich überdrücken und lösen.

Es ist im übrigen vorteilhaft, dem Werkzeug 5 im Funktionsbetrieb ein vorzugsweise steriles flüssiges Kühl- oder Spülmittel oder ein Desinfektionsmittel zuzuführen. Ein solches Mittel kann durch die Medienleitung 7 zugeführt werden, die - die Schnellschlußkupplung 12 durchsetzend - z.B. am vorderen Ende des Handstückschaftes 3 an einer auf die Behandlungsstelle gerichteten Austrittsöffnung 7a austreten kann, wie es in Fig. 1 andeutungsweise dargestellt ist. Im Rahmen der Erfindung ist es auch möglich und vorteilhaft, die Medienleitung 7 längs durch den Handstückschaft 3, längs durch den Werkzeugschaft 5a und im Werkzeugkörper 5b so verlaufen zu lassen, daß sie an einer in der Arbeitsfläche 5c befindlichen Austrittsöffnung austritt. Es ist auch vorteilhaft, gegebenenfalls zusätzlich eine Austrittsöffnung in der als Anlagefläche dienenden Breitseite 22c oder der Breitseite 22d anzuordnen. Mittels der Kühl- oder Spülmittelzuführungsvorrichtung lassen sich auch insbesondere dann, wenn mit dem Werkzeug 5 eine sacklochförmige Präparation eingearbeitet wird, Bearbeitungsspäne aus der sacklochförmigen Präparation wegspülen und entfernen, wobei gleichzeitig die Umfangsflächen des Werkzeugs befeuchtet, gekühlt und gespült werden, wodurch die Leistungsfähigkeit des Werkzeug 5 bei schonender Behandlung verbessert wird. Es ist auch möglich, eine Medienleitung 7 in Form einer Schlauchleitung außenseitig vom Handstück 2 verlaufen zu lassen und mit der Austrittsöffnung 7a oder mit dem Handstückschaft 3 zu verbinden und das Medium im vorbeschriebenen Sinne zur Behandlungsstelle gelangen zu lassen.

Der Handstückschaft 3 ist im Handstück 2 allseitig elastisch schwingbar gelagert. Hierzu können elastisch nachgiebige bzw. komprimierbare Lagerteile, z.B. Lagerringe, dienen, von denen zwei in einem axialen Abstand voneinander angeordnet und andeutungsweise dargestellt sind. Das eine Lagerteil 15 kann radial elastisch nachgiebig sein, während das vorzugsweise vordere Lagerteil 16 radial und axial nachgiebig ist. Aufgrund der elastisch nachgiebigen Ausbildung wird der Handstückschaft 3 durch die Elastizität der Lagerteile 15, 16 im Ruhezustand in eine Vibrations-Mittelstellung zurückgestellt. Der Schwingungserzeuger bzw. Vibrationsantrieb 6 erzeugt hochfrequente kurzhubige Schwingungen im Sinne einer Vibration mit einer vorzugsweise im Schall- oder Ultraschallbereich liegenden Frequenz, wobei die Schwingungen bzw. Amplituden z.B. quer und/oder längs des Handstückschaftes 3 linear gerichtet sein können oder ellipsen- oder kreisförmig umlaufend sein können und zwar jeweils in einer Ebene oder ihre Richtung wechselnd räumlich verlaufen können. Umlaufende Schwingungen haben sich als vorteilhaft erwiesen. Aufgrund der radialen und axialen elastisch nachgiebigen Lagerung des Handstückschaftes 3 stellen sich im Funktionsbetrieb räumliche Schwingungen ein, so daß das Werkzeug 5 in allen Richtungen abrasiv wirksam ist. Die jeweilige Wirkrichtung WR des Werkzeugs 5, in der sich letzteres in das zu bearbeitende Material einsenkt, ist quer, insbesondere rechtwinklig, zur abrasiven Arbeitsfläche 5c des Werkzeugs 5 gerichtet.

Beim vorliegenden Ausführungsbeispiel weist der Vibrationsantrieb eine Frequenz von etwa 4 kHz bis 8 kHz, vorzugsweise etwa 6 kHz, auf, wobei sich im Bereich des Werkzeugs 5 eine Amplitude der vorzugsweise räumlichen Schwingungen von etwa 0,05 mm bis 0,2 mm, insbesondere etwa 0,1 mm, ergibt. Dabei kann die Steuereinrichtung so ausgebildet sein, die sie eine Einstellung der Schwingungsleistung im vorgenannten Bereich oder auch eine Einstellung über diesen Bereich hinaus ermöglicht, so daß gegebenenfalls auch beträchtlich größere Amplituden einstellbar sein können.

Das erfindungsgemäße Instrument 1 eignet sich deshalb besonders gut für unterschiedliche Werkzeuge 5, die als Werkzeug-Sortiment zugeordnet sein können und sich aufgrund unterschiedlicher Form und/oder Größe und/oder Zweckbestimmung voneinander unterscheiden.

Die Haltevorrichtung 4 weist ein Steckloch 17 im Handstückschaft 3 für den vorzugsweise zylindrischen Werkzeugschaft 5a auf und eine Fixiervorrichtung 18 für den Werkzeugschaft 5a, die ihn im Steckloch 17 sichert und starr oder so elastisch nachgiebig sein kann, daß bei Überschreitung einer am Werkzeug 5 in Umfangsrichtung und/oder axial wirksamen Belastungskraft die Fixiervorrichtung 18 selbsttätig löst, wodurch eine Beschädigung oder ein Abbrechen des Werkzeugs 5 vermieden wird. Aus Vereinfachungsgründen ist bei den vorliegenden Ausführungsbeispielen die Fixiervorrichtung 18 als Pfeil dargestellt. Es kann sich um eine in den Handstückschaft 3 radial eingeschraubte Klemmschraube oder eine elastisch vorgespannte Rundung handeln, die jeweils in eine Kalotte (nicht dargestellt) im Werkzeugschaft 5a einfassen.

Der vorzugsweise im Querschnitt runde und insbesondere axial vom Handstück 2 abstehende Handstückschaft 3 kann sich gemäß Fig. 1 gerade erstrecken oder in seinem vorderen Endbereich abgewinkelt sein, z.B. um einen Winkel W1 von etwa 5 bis 30°, insbesondere etwa 15°. Das Steckloch 17 kann sich axial (Fig. 1) oder quer, insbesondere rechtwinklig (Fig.2, 3, 4), im Schaft 3 befinden, wobei der Werkzeugkörper 5b bezüglich des Werkzeugschafts 5a axial (Fig. 1, 2) oder davon quer, z.B. rechtwinklig, abstehend (Fig. 3, 4) angeordnet sein kann. Das erfindungsgemäße Handstück 2 kann auch ein sogenanntes Winkelhandstück sein.

Bereits die vorbeschriebenen Ausgestaltungen des Handstückschafts 2 bzw. Winkelstückschaftes und/oder des Werkzeugs 5 ermöglichen eine Vielzahl von Werkzeugstellungen bezüglich des Instruments 1 bzw. des Handstückschafts 3, so daß auch in schwierigen Bearbeitungsstellungen am zu bearbeitenden Körper, z.B. im Mundraum, eine gute Zugänglichkeit gewährleistet ist. Wie Fig. 4 deutlich zeigt, kann das Werkzeug 5 um die Längsmittelachse des Werkzeugschaftes 5a in Stufen oder stufenlos drehbar und einstellbar in der Haltevorrichtung 4 positioniert werden. Hierdurch wird das Instrument 1 oder das Werkzeug 5 hinsichtlich der Eignung für unzugängliche Bearbeitungsstellen weiter verbessert. Außerdem wird der Vorteil erreicht, daß ein und dasselbe Instrument 1 sich für unterschiedliche Bearbeitungsstellen mit unterschiedlichen Werkzeugen 5 und Werkzeugstellungen eignet.

Gemäß der Erfindung weist das Werkzeug 5 die Form eines Blattes B auf mit einer Schmalseite 21 und zwei davon ausgehenden Breitseiten 22, wobei der Werkzeugkörper 5b sich in der Längsrichtung 23 der Schmalseite 21 länglich erstreckt, wie es bei einem Sägeblatt der Fall ist. Die Schmalseite 21 ist als Arbeitsfläche 5c abrasiv ausgebildet, während die gegebenenfalls nach vorne konvergente Rückenfläche 24, die Breitseiten 22 und die übrigen Schmalseiten 22a, 22b glatt sind. Das Werkzeug 5 eignet sich aufgrund seiner Blattform vorzüglich für schmale Schnitte und zum Schneiden oder Trennen, wobei aufgrund der verhältnismäßig geringen Breite b der Schmalseite 21 bzw. der Dicke des Werkzeugs 5 wenig Zerspanungs- und Trennungsabfall auftritt. Die Breite b kann der eines üblichen Sägeblattes entsprechen, z.B. etwa 1 bis 2 mm betragen.

Die Arbeitsfläche 5c ist mit einer Vielzahl punktförmiger Schneiden besetzt, die auf der gesamten Arbeitsfläche 5c verteilt angeordnet sind. Hierbei kann es sich um geometrisch definierte oder unbestimmte Schneiden handeln. Vorzugsweise handelt es sich um harte adhärente Körner, insbesondere aus Diamant, Feldspat oder Keramik bzw. zahnartige Schneiden aus hartem Material, insbesondere Hartmetall (Sägeblatt). Es ist im Rahmen der Erfindung auch möglich, auf der Arbeitsfläche 5c zahnartige Schneiden, z.B. wie bei einem Sägeblatt, vorzusehen. Der Abstand der Schneiden voneinander ist gleich oder kleiner als die Amplitude der Vibrationsbewegungen bemessen. Hierdurch erweist sich die Arbeitsfläche 5c im Vibrationsbetrieb als abrasiv wirksame Fläche, die einen zu bearbeitenden Gegenstand, z.B. einen Knochen, ein Modell oder einen Zahn, spanabhebend bearbeitet. Dabei verhält sich das Werkzeug 5 prinzipiell wie ein Sägeblatt, da es wie ein Sägeblatt parallel zu seiner Arbeitsfläche 5c am zu bearbeitenden Gegenstand oszillierend bewegt wird. Ein wesentlicher Unterschied des vorliegenden Werkzeugs 5 besteht darin, daß die Bewegungsrichtung der Vibrationsbewegungen unabhängig ist von der Längsrichtung 23 der Arbeitsfläche 5c, wenn bei einer linearen Schwingbewegung diese parallel zur Arbeitsfläche 5c gerichtet ist und bei einer umlaufenden Schwingbewegung die Umlaufebene parallel zur Arbeitsfläche 5c gerichtet ist. Bei einer räumlichen Schwingbewegung ist die Erstreckung der Arbeitsfläche völlig unabhängig. Dies ist dadurch zu erklären, daß die Arbeitsfläche 5c aufgrund ihrer Diamantisierung bzw. ihrer punktförmigen Schneiden bei Schwingbewegungen in einer Ebene in allen Richtungen dieser Ebene schneidend wirksam sein kann und bei einer räumlich umlaufenden Schwingbewegung in jeder Erstreckung wirksam ist, da die räumlichen Schwingbewegungen aufgrund ihrer Bogenform Bewegungskomponenten enthalten, die zumindest teilweise etwa parallel zur Arbeitsfläche 5c gerichtet sind und deshalb die Arbeisfläche 5c immer schabend und somit spanabhebend wirksam ist.

Hieraus folgt, daß das Werkzeug 5 sich in der Längsrichtung 23 der Arbeitsfläche 5c nicht gerade erstrecken muß sondern von dieser Richtung abweichen kann, z.B. winkelförmig, bogenförmig oder ringförmig, wie es beispielhaft die Fig. 6 bis 8 zeigen, wie weiter unten noch beschrieben wird.

Hervorzuheben ist, daß das Werkzeug 5 auch dann abrasiv wirksam und leistungsfähig ist, wenn die Schwingbewegungen quer zu den Breitseiten 22 gerichtet sind. Da die Amplituden der Schwingbewegungen verhältnismäßig klein sind, besteht für das Werkzeug 5 im sich bildenden Schneidspalt ein hinreichend großes Bewegungsspiel, so daß das Werkzeug die Schwingbewegungen auch dann ausführen kann, wenn die Hauptrichtung quer zu den Breitseiten 22 gerichtet ist, an denen das Gewebe des Schneidspaltes seitlich ansteht. Dieses ist zum einen darauf zurückzuführen, daß der Schneidspalt aufgrund der Schwingbewegungen geringfügig größer ist als die Breite b der Arbeitsfläche 5c und außerdem das Gewebe geringfügig nachgiebig ist, insbesondere, wenn es sich beim zu bearbeitenden Gegenstand um einen Knochen oder um das Gewebe eines menschlichen oder tierischen Körpers handelt.

Im folgenden werden die erfindungsgemäßen Ausgestaltungen gemäß den übrigen Figuren beschrieben.

Beim Ausführungsbeispiel nach Fig. 5, bei dem gleiche oder vergleichbare Teile mit gleichen Bezugszeichen versehen sind, ist dem Werkzeug 5 ein Tiefenanschlag 25 zugeordnet, der sich etwa parallel zur Arbeitsfläche 5c erstreckt und/oder in einem Abstand a davon befindet und beim Auftreffen auf den zu bearbeitenden Gegenstand die Einarbeitungsbewegung längs der sich quer zur Arbeitsfläche 5c erstreckenden Einarbeitungs- bzw. Wirkrichtung WR begrenzt. Der Tiefenanschlag 25 kann durch eine rahmenförmige Fassung gebildet sein, insbesondere eine Steckfassung 26, in die ein Werkzeugteil 5.1 einsteckbar ist. Eine solche Steckfassung 26 kann ein Teil 5d des Werkzeugs 5 oder als Adapter 27 ausgebildet sein, wobei das Teil 5d oder der Adapter 27 den Halteschaft 5a aufweisen und mit diesem in der Haltevorrichtung 4 gehalten werden können.

Vorzugsweise ist der Abstand a und somit die durch den Tiefenanschlag 25 bestimmbare Einarbeitungstiefe c verringerbar und vergrößerbar und mittels einer Einstellvorrichtung 28 veränderlich und einstellbar. Hierzu weist das Teil 5d oder der Adapter 27 eine Führung 29 für das Werkzeugteil 5.1 auf, in der es quer zur Arbeitsfläche 5c verschiebbar ist. Bei der vorliegenden Ausgestaltung ist ein scheibenförmiges Werkzeugteil 5.1 vorgesehen, von dessen schmaler Rückenfläche 24 ein Werkzeugschaft 5.1a absteht, auf dem eine Mutter 31 gegen ein vorhandenes Führungsteil 29a schraubbar ist und das Werkzeugteil 5.1 längs der Wirkrichtung WR zu verstellen und einzustellen vermag. Bei der vorliegenden Ausgestaltung ist das Führungsteil 29a ein schmaler, einen rechteckigen Abschnitt des Werkzeugs 5.1 mit geringem Bewegungsspiel aufnehmender Führungsrahmen gebildet, wobei Federelemente 30, z.B. Federdrähte, zwischen dem Werkzeugteil 5.1 und ihm gegenüberliegenden Teilen des Führungsrahmens angeordnet sind. Wie aus Fig. 5 zu entnehmen ist, ist die Führung 29 bezüglich der Längsmittelachse des Handstückschaftes 3 um den Winkel W 1 vorzugsweise entsprechend gekippt angeordnet.

Es ist aus Gründen einer Schmierung der Führung 29 vorteilhaft, die Austrittsöffnung 7a oder eine sich durch den Werkzeugschaft 5.1a erstreckende weiterführende Medienleitung 7 so anzuordnen, daß die Behandlungs- oder Kühlflüssigkeit insbesondere von hinten in die Führung 29 gelangt, von der sie weiter zur Arbeitsfläche 5c fließen kann. Hierbei kann es sich um eine bezüglich des Handstücks extern zugeordnete oder integrierte Kühlmittel-Zuführungsvorrichtung handeln.

Bei den Ausführungsbeispielen nach den Fig. 6 bis 8, bei denen gleiche oder vergleichbare Teile mit gleichen Bezugszeichen versehen sind, ist der Werkzeugkörper 5b bezüglich der Längsrichtung 23 seiner Schmalseite 21 und Arbeitsfläche 5c quer zu den Breitseiten 22 von einer Geraden abweichend geformt, z.B. bogen- oder kreisbogenabschnittförmig oder winkelförmig, wie es Fig. 6 mit durchgezogenen Linien und mit strichpunktierten Linien andeutungsweise zeigt. Bei dieser Ausgestaltung ist der Werkzeugkörper 5b ein blattförmiger Streifen, der entsprechend prismatisch gebogen oder abgewinkelt ist, an der Schmalseite 21 die Arbeitsfläche 5c aufweist und am Rücken 24, vorzugsweise mittig, mit dem Werkzeugschaft 5a verbunden ist. Bei dieser Ausgestaltung erstreckt sich die Arbeitsfläche 5c entsprechend bogen- oder winkelförmig, wobei sie sich in einer quer zum Werkzeugschaft 5a verlaufenden Ebene erstrecken kann oder eine von dieser Ebene abweichende Form aufweisen kann. Die übrigen beiden Schmalseiten 22e, 22d können parallel oder keilförmig zueinander oder zur Wirkrichtung WR ausgebildet sein.

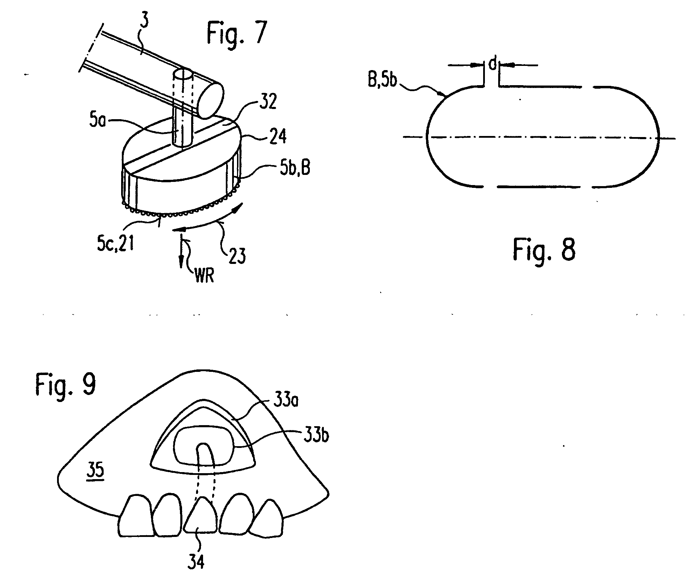

Bei der Ausgestaltung nach Fig. 7, bei der gleiche oder vergleichbare Teile mit gleichen Bezugszeichen bezeichnet sind, ist der Werkzeugkörper 5b in Umfangsrichtung geschlossen bzw. rohr- oder rahmenförmig, ausgebildet, wobei die Darstellung einen Werkzeugkörper 5b zeigt, dessen Streifen oder Blatt hohlzylindrisch geformt oder gebogen ist. In vergleichbarer Weise kann der Werkzeugkörper 5b längs des Werkzeugschaftes 5a gesehen eine rechtwinklige oder dreieckige hohle Querschnittsform gegebenenfalls mit gerundeten Ecken aufweisen. Der Werkzeugschaft 5a kann dabei am der umlaufenden Arbeitsfläche 5c abgewandten Rücken 24 des Blattes und somit exzentrisch oder an einer transversal angeordneten Speiche 32 zentral angeordnet sein, was sich auch bei einer Ausgestaltung nach Fig. 6 verwirklichen läßt.

Wie Fig. 8 zeigt, muß der Werkzeugkörper 5b in Umfangsrichtung nicht durchgehend ausgebildet sein, sondern er kann aus geraden und/oder ungeraden Abschnitten bzw. Segmenten bestehen, die einen sich in der Umfangsrichtung erstreckenden Abstand d voneinander aufweisen können. Mit einem in Umfangsrichtung geschlossenen Werkzeugkörper 5b läßt sich ein Loch in einem Zuge einarbeiten bzw. -schneiden und durch Abtrennung in seinem Grundbereich entfernen. Bei der Ausgestaltung nach Fig. 8 sind die verbleibenden Stege im Bereich der Abstände d durch manuelle Nacharbeit zu trennen. Bei der Benutzung von Werkzeugkörpern 5b, die in Umfangsrichtung nicht geschlossen sind, z.B. winkelförmig oder U-förmig geformt sind, kann durch Einschneiden in den Gegenstand in mehreren Gängen und ein entsprechendes Versetzen nach jedem Schnitt ein zu entfernender Abschnitt des zu bearbeitenden Gegenstandes ausgearbeitet und entfernt werden.

Es ist im übrigen bei den Ausgestaltungen nach den Fig. 6 bis 8 möglich und je nach Einsatzfall vorteilhaft, den segment- oder ring- bzw. hülsenförmigen Werkzeugkörper 5b nach vorne konvergent bzw. im Sinne eines Pyramiden- oder Kegelstumpfes auszubilden, wodurch sich reponierbare Körperteile, z.B. sog. Knochendeckel, ausarbeiten und wieder einsetzen lassen. Fig. 9 zeigt eine vorbeschriebene Operation im Rahmen einer Zahnwurzelspitzenresektion, bei der zunächst ein größerer Ring- oder Winkelschnitt 33a eingearbeitet und das entsprechende Körperteil entfernt oder abgeklappt wird und dann ein kleinerer Ringschnitt 33b im Bereich der Zahnwurzelspitze des vorhandenen Zahns 34 eingearbeitet und somit die Wurzelspitze freigelegt wird.

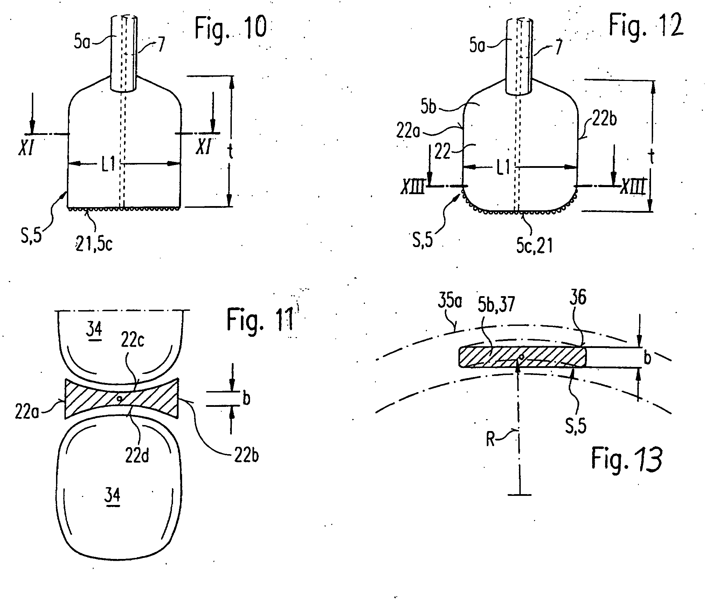

Bei der erfindungsgemäßen Ausgestaltung läßt sich die mit L bezeichnete Länge des Werkzeugskörpers 5b um ein mehrfaches größer als seine Tiefe t ausbilden, wie es z.B. die vorbeschriebenen Fig. 1 bis 7 zeigen, oder die Länge L kann etwa der Tiefe t entsprechen oder kleiner als die Tiefe t sein, wodurch der Werkzeugkörper 5b die Form eines Spatels S mit vorzugsweise parallel zueinander verlaufenden Breitseiten 22c, 22d und Schmalseiten 22a, 22b erhält S. Fig. 10 bis 13.

Ein erfindungsgemäßes Werkzeug 5 in der vorbeschriebenen oder einer ähnlichen Spatelform eignet sich auch für paradontal chirurgische Kronenverlängerungen , wie es die Fig. 10 bis 11 zeigen, bei denen gleiche oder vergleichbare Teile mit gleichen Bezugszeichen versehen sind, weist der spatelförmige Werkzeugkörper 5b an beiden Breitseiten 22c, 22d konkave bzw. hohlzylinderabschnittförmige glatte Anlageflächen auf, wobei nur die Arbeitsfläche 5c abrasiv sein und sich gerade erstrecken oder etwas konvex geformt sein kann, um im Bereich einer Kronenverlängerung vorhandenes Material des Zahnfleisches 34a oder auch des Kieferknochens 35 zu entfernen.

Bei bekannten Knochenbettpräparationen mit rotierenden Werkzeugen ist es nur möglich rotationssymmetrische Aufnahmeöffnungen für zylinder- konus- oder schraubenförmige Implantate zu präparieren. Der Erfindung liegt die Erkenntnis zugrunde, daß längs des Zahnbogens längliche Präparationen aus anatomischen Gründen (Kiefernhöhle, Nervenkanäle) günstiger sind, und daß sich ein erfindungsgemäßes spatelförmiges Werkzeug 5 zur Ausarbeitung solcher Knochenbettpräparationen sehr gut eignet. Aufgrund der länglichen Querschnittsform kommt das erfindungsgemäße Werkzeug 5 und eine damit ausgearbeitete Knochenbettpräparation den vorgenannten, aus den anatomischen Gegebenheiten resultierenden Forderungen entgegen, wobei eine für die Kreisbogenform des Kiefers 35 günstige Form und Position der Knochenbettpräparation erreicht wird und Nervenkanäle unbeeinträchtigt bleiben.

Hierzu eignet sich ein spatelförmiges oder keilförmiges Werkzeug 5 gemäß Fig. 12 und 13 besonders gut, bei denen gleiche oder vergleichbare Teile mit gleichen Bezugszeichen versehen sind. Die Spatelform kann bezüglich der Abmessung L1 parallel sein oder sich zum freien bzw. vorderen Ende hin keilförmig verjüngen.

Eine entsprechende Querschnittsform kann auch das Implantat 37 aufweisen.

In Fig. 13 ist die Querschnittsform des Werkzeugkörpers 5b oder Implantats 37 in Relation eines Kieferknochenbogens 35a als sich gerade erstreckende Querschnittsform und als sich gekrümmt (strichpunktiert angedeutet) erstreckende Querschnittsform dargestellt. Die Einarbeitung der Aufnahmeöffnung 36 für ein zugehöriges Implantat, das hier eine plättchenförmige bzw. leistenförmige Querschnittsform aufweist, kann in einem Zuge nur mit dem erfindungsgemäßen Werkzeug 5 eingearbeitet werden, oder es können die seitlichen Enden der Aufnahmeöffnung 36 durch ein rotierendes Werkzeug entsprechender Querschnittsgröße vorpräpariert werden und dann der dazwischen liegende Bereich mit einem erfindungsgemäßen Werkzeug 5 zum z.B. schlitzförmigen Aufnahmeöffnungsquerschnitt ergänzt werden.

Eine vorbeschriebene blattartige Querschnittsform des Werkzeugkörpers 5b und/oder des Implantats 37 ergibt auch eine vorteilhafte Anpassung an die Zahnwurzelquerschnitte, die in bukko-lingualer Richtung längsoval sind, insbesondere im Frontzahnbereich des Ober- und Unterkiefers.

Nachfolgend werden weitere durch die Erfindung erzielbare Vorteile zusammenfassend angeführt.

Bei bekannten drehangetriebenen Bohrern oder Fräsern zur Knochenentfernung, z.B. bei Weisheitszahnentfernungen, ist wegen der beträchtlichen Festigkeit des Zerspanungsgegenstandes ein manuelles kontrolliertes Führen nur erschwert möglich, weil das Werkzeug dazu neigt, aus einer beabsichtigten Führung zu verlaufen. Bei der erfindungsgemäßen Ausgestaltung läßt sich das Werkzeug nicht nur gezielt am zu bearbeitenden Gegenstand ansetzen, sondern ein einmal angebrachter Ansatz läßt sich paßgenau weiter einbringen, ohne das besondere manuelle Anstrengungen vorgenommen werden müssen, um ein Verlaufen zu verhindern.

Bei einem erfindungsgemäßen Werkzeug 5 sind Beschädigungen oder Verletzungen angrenzender Zähne vermieden, insbesondere bei marginaler/interdentaler Knochenentfernung bei parodontal-chirurgischer Kronenverlängerung.

Im Gegensatz zu bekannten Knochensägen, die nur in der Richtung der Hin- und Herbewegung funktionsfähig sind, sind beim erfindungsgemäßen Werkzeug auch von der Hin- und Herbewegung abweichende Formen der Bewegung des Werkzeugkörpers 5b möglich.

Bei den Ausgestaltungen gemäß Fig. 10 bis 13 kann der Werkzeugschaft 5a in der Längsrichtung der Spatelform, d.h. etwa parallel zur Arbeitsfläche 5c oder quer dazu angeordnet sein.

Bei allen Ausführungsbeispielen kann die Breite b des Werkzeugkörpers 5b bzw. Blattes B oder Spatels S etwa 1mm bis 3mm, vorzugsweise etwa 2mm, betragen.

## Patentansprüche

1. Medizinisches oder dentalmedizinisches Instrument (1) zur spanabhebenden Bearbeitung von Körpergewebe oder einem Ersatzstoff, mit
- einem länglichen, insbesondere geraden oder winkelförmigen, Handstück (2),
- einer im vorderen Endbereich des Handstücks (2) angeordneten Haltevorrichtung (4),
- und einem Werkzeug (5) mit einem Werkzeugschaft (5a) und einem damit verbundenen Werkzeugkörper (5b), dessen Werkzeugschaft (5a) durch die Haltevorrichtung (4) mit dem Handstück (2) lösbar oder unlösbar verbunden ist,
- wobei im Handstück (2) ein Vibrationsantrieb (6) für das Werkzeug (5) angeordnet ist, der hochfrequente, in wenigstens einer Ebene hin und her oder umlaufende oder räumliche Schwingbewegungen auf das Werkzeug (5) überträgt,
- und wobei der Werkzeugkörper (5b) eine abrasive Arbeitsfläche (5c) aufweist,
**dadurch gekennzeichnet,**
**daß** der Werkzeugkörper (5b) durch ein Blatt (B) gebildet ist, das
die abrasive Arbeitsfläche (5c) an einer Schmalseite (21) aufweist.

2. Werkzeug (5) für ein medizinisches oder dentalmedizinisches Instrument (1) zur spanabhebenden Bearbeitung von Körpergewebe oder einem Ersatzstoff,
- wobei das Werkzeug (5) mit einem Werkzeugkörper (5b) und einem damit verbundenen Werkzeugschaft (5a) ausgebildet ist, der durch eine im vorderen Endbereich eines Handstücks (2) eines medizinischen oder dentalmedizinischen Instruments (1) angeordnete Haltevorrichtung (4) mit dem Handstück (2) verbindbar ist,
- und wobei der Werkzeugkörper (5b) eine abrasive Arbeitsfläche (5c) aufweist,
**dadurch gekennzeichnet,**
**daß** der Werkzeugkörper (5b) durch ein Blatt (B) gebildet ist, das
die abrasive Arbeitsfläche (5c) an einer Schmalseite (21) aufweist.

3. Instrument nach Anspruch 1 oder Werkzeug nach Anspruch 2,
**dadurch gekennzeichnet,**
**daß** der Werkzeugkkörper (5b) die abrasive Arbeitsfläche (5c) nur an der Schmalseite (21) aufweist.

4. Instrument nach Anspruch 1 oder 3 oder Werkzeug nach Anspruch 2 oder 3,
**dadurch gekennzeichnet,**
**daß** die Arbeitsfläche (5c) eben ist oder in ihrer Querrichtung konvex gerundet ist.

5. Instrument nach einem der Ansprüche 1 oder 3 bis 4 oder Werkzeug nach einem der Ansprüche 2 bis 4,
**dadurch gekennzeichnet,**
**daß** das Blatt (B) eine von seiner Längsrichtung (23) quer zu seinen Breitseiten (22) abweichende Form aufweist.

6. Instrument oder Werkzeug nach Anspruch 5,
**dadurch gekennzeichnet,**
**daß** die Form winkelförmig oder gerundet bzw. gekrümmt ist.

7. Instrument nach Anspruch 5 oder 6 oder Werkzeug nach Anspruch 5 oder 6,
**dadurch gekennzeichnet,**
**daß** die Form ringförmig ist, wobei die Querschnittsform des Werkzeugkörpers (5b) in der Achsrichtung der ringförmigen Form vorzugsweise prismatisch ist oder sich zum vorderen Ende verjüngt.

8. Instrument oder Werkzeug nach Anspruch 7,
**dadurch gekennzeichnet,**
**daß** die Querschnittsform des Ringes rund oder unrund oder oval oder dreieckig oder rechteckig oder nierenförmig ist oder länglich tailliert ist, z.B. im Sinne einer taillierten Niere.

9. Instrument nach einem der Ansprüche 1 oder 3 bis 8 oder Werkzeug nach einem der Ansprüche 2 bis 8,
**dadurch gekennzeichnet,**
**daß** der Werkzeugschaft (5a) längs oder quer zur die Arbeitsfläche (5c) aufweisenden Schmalseite (21) angeordnet ist.

10. Instrument nach einem der Ansprüche 1 oder 3 bis 9,
**dadurch gekennzeichnet,**
**daß** mehrere Werkzeuge (5) unterschiedlicher Form und/oder Größe vorgesehen sind.

11. Instrument nach einem der Ansprüche 1 oder 3 bis 10,
**dadurch gekennzeichnet,**
**daß** der Vibrationsantrieb (6) hinsichtlich einer Vergrößerung oder Verringerung der Leistung und/oder der Frequenz und/oder der Größe der Amplituden der Schwingungen einstellbar ist.

12. Instrument nach einem der Ansprüche 1 oder 3 bis 11,
**dadurch gekennzeichnet,**
**daß** dem Werkzeugkörper (5b) ein Anschlag (25) zur Begrenzung der Einarbeitungstiefe (c) am Handstück (2) oder Werkzeug (5) zugeordnet ist.

13. Instrument nach Anspruch 12,
**dadurch gekennzeichnet,**
**daß** der Abstand (c) des Anschlags (25) von der Arbeitsfläche (5c) durch eine Einstellvorrichtung (28) verringerbar und/oder vergrößerbar ist.

14. Instrument nach einem der Ansprüche 1 oder 3 bis 13,
**dadurch gekennzeichnet**,
die Breite (b) des Blattes (B) etwa 1 bis 3mm, insbesondere etwa 2mm, beträgt.

15. Instrument nach einem der Ansprüche 1 oder 3 bis 14,
**dadurch gekennzeichnet,**
**daß** es wenigstens eine Medienleitung (7) aufweist, die am vorderen Ende des Handstücks (2) an einer Austrittsöffnung (38) mündet oder sich längs durch das Werkzeug (5) erstreckt und an einer in der schmalseitigen und/oder umfangsseitigen Arbeitsfläche (5c) angeordneten Austrittsöffnung (38a) mündet.

## Claims

1. Medical or dental-medical instrument (1) for the material-removing working of body tissue or a substitute material, having
- an elongate, in particular straight or angle-shaped, handpiece (2),
- a holding device (4) arranged in the forward end region of the handpiece (2),
- and a tool (5), having a tool shaft (5a) and a tool body (5b) connected therewith, the tool shaft (5a) of which is releasably or non-releasably connected with the handpiece (2) by means of the holding device (4),
- there being arranged in the handpiece (2) a vibration drive (6) for the tool (5), which transfers to the tool (5) high frequency oscillating movements, back and forth in at least one plane or orbital or three-dimensional,
- the tool body (5b) having an abrasive working surface (5c),
**characterised in that**,
the tool body (5b) is formed by means of a lamina (B) which has the abrasive working surface (5c) at a narrow edge (21).

2. Tool (5) for a medical or dental-medical instrument (1) for the material-removing working of body tissue or a substitute material,
- the tool (5) having a tool shaft (5a) and a tool body (5b) connected therewith, the tool shaft (5a) being connectable with a handpiece (2) of a medical or dental-medical instrument by means of a holding device (4) arranged in the forward end region of the handpiece (2)
- and the tool body (5b) having an abrasive working surface (5c),
**characterised in that**,
the tool body (5b) is formed by means of a lamina (B) which has the abrasive working surface (5c) at a narrow edge (21).

3. Instrument according to claim 1 or tool according to claim 2,
**characterised in that**,
the tool body (5b) has the abrasive working surface (5c) only at the narrow edge (21).

4. Instrument according to claim 1 or 3 or tool according to claim 2 or 3,
**characterised in that**,
the working surface (5c) is flat, or in its transverse direction is convexly rounded.

5. Instrument according to any of claims 1 or 3 to 4 or tool according to any of claims 2 or 4,
**characterised in that**,
the lamina (B) has a shape deviating from its longitudinal direction (23) transversely to its broad faces (22).

6. Instrument or tool according to claim 5,
**characterised in that**,
the shape is angle-shaped or rounded or curved.

7. Instrument according to claim 5 or 6 or tool according to claim 5 or 6,
**characterised in that**,
the shape is ring-form, whereby the cross-sectional shape of the tool body (5b), in the axial direction of the ring-form shape is preferably prismatic or tapers towards the forward end.

8. Instrument or tool according to claim 7,
**characterised in that**,
the cross-sectional shape of the ring is circular or non-circular or oval or triangular or rectangular or kidney-shaped or has a longitudinal waist, e.g. in the sense of a waisted kidney.

9. Instrument according to any of claims 1 or 3 to 8 or tool according to any of claims 2 to 8,
**characterised in that**,
the tool shaft (5a) is arranged longitudinally (e.g. Fig. 1) or transversely to the narrow edge (21) having the working surface (5c).

10. Instrument according to any of claims 1 or 3 to 9,
**characterised in that**,
there are provided a plurality of tools (5) of differing shape and/or size.

11. Instrument according to any of claims 1 or 3 to 10,
**characterised in that**,
the vibration drive (6) is settable with regard to an increase or reduction of the power and/or the frequency and/or the size of the amplitudes of the oscillations.

12. Instrument according to any of claims 1 or 3 to 11,
**characterised in that**,
the tool body (5b) has associated therewith a stop (25) on the handpiece (2) or tool (5) for limitation of the working in depth (c).

13. Instrument according to claim 12,
**characterised in that**,
the spacing (c) of the stop (25) from the working surface (5c) can be decreased and/or increased by means of a setting device (25).

14. Instrument according to any of claims 1 or 3 to 13,
**characterised in that**,
the width (b) of the lamina (B) is about 1 to 3 mm, in particular about 2 mm.

15. Instrument according to any of claims 1 or 3 to 14,
**characterised in that**,
it has at least one media line (7) which opens at the forward end of the handpiece (2) at an exit opening (38) or extends longitudinally through the tool (5) to an exit opening (38a) arranged in the narrow edge and/or circumferential working surface (5c).

## Revendications

1. Instrument (1) médical ou de médecine dentaire pour le traitement par enlèvement de copeaux de tissu de corps ou d'un produit de substitution, comprenant
- une pièce à main (2) allongée, en particulier droite ou en forme d'angle,
- un dispositif de retenue (4) disposé dans la zone d'extrémité avant de la pièce à main (2),
- et un outil (5) avec une tige d'outil (5a) et un corps d'outil (5b) relié à celle-ci, dont la tige d'outil (5a) est reliée de façon détachable ou indétachable par le dispositif de retenue (4) à la pièce à main (2),
- une commande de vibration (6) pour l'outil (5) étant disposée dans la pièce à main (2), qui transmet des mouvements de vibration de haute fréquence, faisant des mouvements d'aller et retour dans au moins un plan ou de rotation ou dans l'espace, à l'outil (5),
- et le corps d'outil (5b) présentant une surface de travail (5c) abrasive,
**caractérisé en ce que**,
le corps d'outil (5b) est formé par une lame (B), qui présente la surface de travail (5c) abrasive sur un côté étroit (21).

2. Outil (5) pour un instrument (1) médical ou de médecine dentaire pour le traitement par enlèvement de copeaux de tissu de corps ou d'un produit de substitution,
- l'outil (5) étant conçu avec un corps d'outil (5b) et une tige d'outil (5a) reliée à celui-ci, qui peut être reliée à la pièce à main (2) par un dispositif de retenue (4) disposé dans la zone d'extrémité avant d'une pièce à main (2) d'un instrument (1) médical ou de médecine dentaire,
- et le corps d'outil (5b) présentant une surface de travail (5c) abrasive,
**caractérisé en ce que**
le corps d'outil (5b) est formé par une lame (B), qui présente la surface de travail (5c) abrasive sur un côté étroit (21).

3. Instrument selon la revendication 1 ou outil selon la revendication 2,
**caractérisé en ce que**
le corps d'outil (5b) présente la surface de travail (5c) abrasive seulement sur le côté étroit (21).

4. Instrument selon la revendication 1 ou 3 ou outil selon la revendication 2 ou 3,
**caractérisé en ce que**
la surface de travail (5c) est plane ou arrondie de façon convexe dans sa direction transversale.

5. Instrument selon l'une quelconque des revendications 1 ou 3 jusqu'à 4 ou outil selon l'une quelconque des revendications 2 à 4,
**caractérisé en ce que**
la lame (B) présente une forme divergente de sa direction longitudinale (23) transversalement à ses côtés larges (22).

6. Instrument ou outil selon la revendication 5,
**caractérisé en ce que**
la forme est en forme d'angle ou arrondie ou incurvée.

7. Instrument selon la revendication 5 ou 6 ou outil selon la revendication 5 ou 6,
**caractérisé en ce que**
la forme est annulaire, la forme de section du corps d'outil (5b) étant de préférence prismatique dans la direction d'axe de la forme annulaire ou se rétrécit vers l'extrémité avant.

8. Instrument ou outil selon la revendication 7,
**caractérisé en ce que**
la forme de section de la bague est ronde ou ovalisée ou ovale ou triangulaire ou rectangulaire ou en forme de rein ou est taillée de façon allongée, par exemple dans le sens d'un rein taillé.

9. Instrument selon l'une quelconque des revendications 1 ou 3 jusqu'à 8 ou outil selon l'une quelconque des revendications 2 jusqu'à 8,
**caractérisé en ce que**
la tige d'outil (5a) est disposée le long ou transversalement au côté étroit (21) présentant la surface de travail (5c).

10. Instrument selon l'une quelconque des revendications 1 ou 3 jusqu'à 9,
**caractérisé en ce que**
plusieurs outils (5) de forme et/ou de grandeur différentes sont prévus.

11. Instrument selon l'une quelconque des revendications 1 ou 3 jusqu'à 10,
**caractérisé en ce que**
la commande de vibration (6) peut être réglée en ce qui concerne une augmentation ou une réduction de la puissance et/ou de la fréquence et/ou de la grandeur des amplitudes des vibrations.

12. Instrument selon l'une quelconque des revendications 1 ou 3 jusqu'à 11,
**caractérisé en ce que**
une butée (25) pour limiter la profondeur de cavité (c) sur la pièce à main (2) ou sur l'outil (5) est attribuée au corps d'outil (5b).

13. Instrument selon la revendication 12,
**caractérisé en ce que**
la distance (c) de la butée (25) à la surface de travail (5c) peut être réduite et/ou agrandie par un dispositif de réglage (28).

14. Instrument selon l'une quelconque des revendications 1 ou 3 jusqu'à 13,
**caractérisé en ce que**
la largeur (b) de la lame (B) est d'environ 1 à 3 mm, en particulier d'environ 2 mm.

15. Instrument selon l'une quelconque des revendications 1 ou 3 jusqu'à 14,
**caractérisé en ce que**
il présente au moins une conduite de fluide (7), qui débouche sur l'extrémité avant de la pièce à main (2) sur une ouverture de sortie (38) ou s'étend dans le sens de la longueur à travers l'outil (5) et débouche sur une ouverture de sortie (38a) disposée dans la surface de travail (5c) côté étroit et/ou côté périphérique.
